# EUROPÄISCHE PATENTSCHRIFT

(11) **EP 2 396 058 B1**
(45) Veröffentlichungstag und Bekanntmachung des Hinweises auf die Patenterteilung: **13.03.2019**
(21) Anmeldenummer: 10702244.4
(22) Anmeldetag: 22.01.2010
(51) Int. Cl.: A61M 5/20, A61M 5/46

(54) **ELEKTROMECHANISCHE INJEKTIONSVORRICHTUNG**
ELECTROMECHANICAL INJECTION APPARATUS
DISPOSITIF D'INJECTION ÉLECTROMÉCANIQUE

(30) Priorität: 13.02.2009 DE 202009001836 U
(43) Veröffentlichungstag der Anmeldung: 21.12.2011
(73) Patentinhaber: Dieter Hölzle Technik-Projekte GmbH, 75392 Deckenpfronn (DE)
(72) Erfinder: RENZ, Andreas, 72172 Sulz (DE); WEBER, Wilfried, 72296 Schopfloch (DE)
(74) Vertreter: Wacker, Jost Oliver
(86) Internationale Anmeldenummer: PCT/EP2010/000372
(87) Internationale Veröffentlichungsnummer: WO 2010/091774

(56) Entgegenhaltungen:
- EP-A1- 1 518 575
- WO-A1-2007/033638
- DE-U1- 8 431 533
- US-A- 4 921 487
- US-A1- 2008 015 512

## Beschreibung

Die Erfindung betrifft eine elektromechanische Injektionsvorrichtung für medizinische Anwendungen nach dem Oberbegriff des Anspruchs 1. Diese Injektionsvorrichtung weist ein Trägergehäuse auf, in dem ein Injektionsgerät, wie eine Spritze oder eine Karpule, mit wenigstens einem auspressbaren Injektionsflüssigkeitsbehälter einsetzbar ist. Zudem weist die Injektionsvorrichtung eine zur Aktivierung des Injektionsgerätes entlang einer Injektionsrichtung antreibbare Betätigungsvorrichtung auf, die zur Ausführung wenigstens eines Einstechhubes und eines Injektionshubes sowohl von einer mechanischen Kraftspeichereinrichtung als auch von einer elektrischen Transporteinrichtung beaufschlagbar ist.

Aus der EP 0 375 584 B1 ist eine Injektionsvorrichtung für medizinischen und tierärztlichen Gebrauch bekannt. Diese weist eine Aufnahme zur Unterbringung einer Spritze und einen elektrisch angetriebenen Schieber auf, mittels dem an der Spritze selbsttätig ein Injektionshub vorgenommen werden kann. Der Schieber weist dabei ein Federelement auf, mittels dem ein Spritzenkolben der Spritze in einer Endstellung des Schiebers unter Vorspannung gehalten werden kann.

Nachteilig an einer derartigen Injektionsvorrichtung ist, dass der zum Antrieb des Schiebers benötigte Elektromotor auf einen bestimmten Lastfall ausgelegt werden muss, der sicherstellt, dass beispielsweise im Falle einer fertigungstoleranzbedingten Verengung einer verwendeten Kanüle die verwendete Spritze oder Karpule nicht zerstört wird. Eine derartige Auslegung des Elektromotors kann aber wiederum dazu führen, dass dieser häufig stehen bleibt und beschädigt wird, wodurch die Injektionsvorrichtung insgesamt häufig aussetzen oder vollständig unbrauchbar werden kann. Zudem ist es bei Verwendung eines Elektromotors zur Durchführung des Injektionshubes nicht oder nur mit einer sehr aufwendigen Steuerung möglich, einen bestimmten gleich bleibenden Druck auf das Injektionsgerät auszuüben, und zwar unabhängig davon, ob die Kanüle verengt oder verstopft ist.

DE 8431533U1 beschreibt eine Infusionspumpe, bei der eine durch eine Feder angetriebene Betätigungseinrichtung zur Beaufschlagung eines Spritzenkolbens vorgesehen ist. Die Betätigungseinrichtung ist dabei stangenförmig ausgebildet und trägt ein Schneckenrad, das mit einer Schneckenwelle zusammen wirkt, die durch einen elektrischen Antrieb in Rotation versetzt werden kann. Die Haftreibung zwischen dem Schneckenrad und der Schneckenwelle ist bei Stillstand der Schnecknwelle ausreichend, um die Betätigungseinrichtung entgegen der Vorspannung der Feder in einer Ausgangsstellung zu halten. Durch Antrieb der Schneckenwelle wird die Haftreibung aufgehoben, wobei bei der nun herrschenden Gleitreibung die Bewegung der Betätigungseinrichtung frei gegeben ist.

US 4,921,487 beschreibt eine Injektionsvorrichtung zur kontinuierlich wiederkehrenden Verabreichung einer konstanten Dosis eines Medikaments. Zudem ermöglicht die Vorrichtung auch die momentane Verabreichung einer größeren Dosis durch manuelle Beaufschlagung. Für die kontinuierliche Verabreichung ist in der zweiten und dritten Ausführungsform jeweils ein elektrischer Motor vorgesehen, der schrittweise arbeitet und dabei auf Regulierungsmittel beziehungsweise auf ein Uhrwerk einwirkt, wodurch eine Antriebseinrichtung, die allein von mechanischer Energie beaufschlagt ist, wiederkehrend kurzzeitig frei gegeben wird, um eine Injektionsbewegung auzuführen.

Die Aufgabe der Erfindung ist es, bei einer elektromechanischen Injektionsvorrichtung die genannten Nachteile zu vermeiden und bei komfortabler Bedienbarkeit eine zuverlässige Funktionsweise und hohe Langlebigkeit zu ermöglichen.

Diese Aufgabe wird durch eine Injektionsvorrichtung mit den Merkmalen des Anspruchs 1 gelöst. Dabei ist die Betätigungsvorrichtung in Injektionsrichtung, vorzugsweise über die gesamte Hublänge durch die Antriebskraft einer mechanischen Kraftspeichereinrichtung angetrieben. Die elektrische Transporteinrichtung bildet hierbei eine Geschwindigkeitsbegrenzung, die während einer Injektion zur Begrenzung einer durch die Kraftspeichereinrichtung erzielten Hubgeschwindigkeit entgegengesetzt zur Antriebskraft wirkt. Auf diese Weise ist es möglich, alle für die Injektion eines Arzneimittels benötigten Hubbewegungen in Injektionsrichtung allein mittels der mechanischen Kraftspeichereinrichtung auszuführen. Hierbei werden die für die Ausführung eines Einstechhubes, eines Injektionshubes und gegebenenfalls eines vorgeschalteten Mischhubes erforderlichen Antriebskräfte allein durch die Kraftspeichereinrichtung auf das Injektionsgerät aufgebracht. Die elektrische Transporteinrichtung wird dagegen während der Injektion lediglich dazu verwendet, in entgegen gesetzter Richtung zur Antriebskraft am Injektionsgerät anzugreifen, um die durch die Kraftspeichereinrichtung erzielten Hubgeschwindigkeiten zu begrenzen beziehungsweise zu steuern. Da somit der Antrieb der Betätigungsvorrichtung in Injektionsrichtung allein über die Kraftspeichereinrichtung erfolgt, muss auch lediglich diese so ausgelegt werden, dass eine Beschädigung des Injektionsgerätes ausgeschlossen werden kann. Der Elektroantrieb, dessen Antriebskräfte auf diese Weise nicht auf das Injektionsgerät einwirken, kann unabhängig hiervon ausgelegt werden, wodurch die Injektionsvorrichtung insgesamt wesentlich zuverlässiger arbeitet und eine größere Langlebigkeit erzielt. Zudem kann durch die alleinige Beaufschlagung des Injektionsgerätes in Injektionsrichtung mittels der mechanischen Kraftspeichereinrichtung auch bei verengter oder verstopfter Kanüle des Injektionsgerätes ein gleich bleibender Druck auf das Injektionsgerät und das darin enthaltene Medikament gewährleistet werden.

Vorteilhafterweise weist die Kraftspeichereinrichtung ein zwischen der Betätigungsvorrichtung und dem Trägergehäuse eingespanntes Federmittel auf, wodurch mit einfachen und kostengünstigen Mitteln eine zuverlässige Kraftbeaufschlagung der Betätigungsvorrichtung in Injektionsrichtung über die gesamte Hublänge gewährleistet werden kann.

Dabei ist es günstig, wenn die Betätigungsvorrichtung von der elektrischen Transporteinrichtung ausschließlich entgegen der Injektionsrichtung beaufschlagbar ist. Auf diese Weise wird sichergestellt, dass die Betätigungsvorrichtung in Injektionsrichtung allein durch die mechanische Kraftspeichereinrichtung beaufschlagt ist, die somit als mechanische Transporteinrichtung wirkt. Die elektrische Transporteinrichtung kann somit während eines Injektionsvorganges der Antriebskraft lediglich entgegen wirken, um die Hubgeschwindigkeit zu limitieren. Hierdurch ist keine besondere Leistungsbegrenzung des elektrischen Antriebs notwendig, da dieser in keiner sicherheitsrelevanten Weise auf die Betätigungsvorrichtung beziehungsweise auf das Injektionsgerät einwirkt.

In einer besonders vorteilhaften Ausführungsform kann die Betätigungsvorrichtung durch die elektrische Transporteinrichtung entgegen der Injektionsrichtung von einer Injektionsposition in eine Ausgangsposition verbracht werden. Hierdurch kann die elektrische Transporteinrichtung dazu verwendet werden, die Betätigungsvorrichtung beispielsweise nach einer verabreichten Injektion selbsttätig wieder in eine Ausgangsstellung zu verbringen, in der ein neues Injektionsgerät eingesetzt und mit diesem ein neuer Injektionsvorgang durchgeführt werden kann. Auf diese Weise ist es möglich die Injektionsvorrichtung nach jeder erfolgten Anwendung ohne besonderen Kraftaufwand wieder in einen betriebsbereiten Zustand zu versetzen, was eine besonders komfortable Handhabung und insbesondere auch die eigenständige Verwendung der Injektionsvorrichtung durch gesundheitlich gehandicapte Benutzer ermöglicht.

Vorteilhafterweise weist die Betätigungsvorrichtung einen Einstechschlitten mit einer Aufnahme für das Injektionsgerät sowie einen relativ zum Einstechschlitten verlagerbaren Injektionsschlitten auf, der einen Betätigungsstößel für die Beaufschlagung eines Stößels des Injektionsgerätes aufweist, wobei der Einstechschlitten und der Injektionsschlitten von der mechanischen Kraftspeichereinrichtung und der elektrischen Transporteinrichtung wenigstens zur Durchführung eines Einstechhubes, eines Injektionshubes und eines Rückholhubes ansteuerbar sind. Hierdurch ist eine genaue Steuerung der Betätigungsvorrichtung während aller wesentlichen Hubbewegungen der Injektionsvorrichtung möglich, um beispielsweise ein für eine Injektionsanwendung besonders geeignetes Geschwindigkeitsprofil vorab festlegen und während de Anwendung durchlaufen zu können. Vorteilhafterweise ist der Injektionsschlitten von der mechanischen Kraftspeichereinrichtung und der elektrischen Transporteinrichtung zusätzlich zur Durchführung eines dem Einstechhub vorgeschalteten Mischhubes ansteuerbar. Hierdurch ist es möglich, die Injektionsvorrichtung auch für Injektionsgeräte zu verwenden, die wenigstens zwei Komponenten eines zu injizierenden Medikamentes getrennt voneinander bereithalten, die vor dem eigentlichen Injektionsvorgang zunächst miteinander vermischt werden müssen.

Zudem ist es günstig, wenn die elektrische Transporteinrichtung eine durch eine Spindel antreibbare Transportmutter aufweist, die zum Transport der Betätigungsvorrichtung während des Rückholhubes gegen einen Betätigungsanschlag an einer der Injektionsrichtung zugewandten Seite der Betätigungsvorrichtung anlegbar ist. Hierdurch ist eine besonders exakte Positions- beziehungsweise Bewegungssteuerung der Betätigungsvorrichtung möglich. Zudem können auf diese Weise auch relativ große Rückholkräfte problemlos auf die Betätigungsvorrichtung aufgebracht werden, um diese entgegen der Antriebskraft der mechanischen Kraftspeichereinrichtung in die Ausgangsposition zurück zu transportieren.

Vorteilhafterweise ist die Betätigungsvorrichtung zudem zur Steuerung der Geschwindigkeit einer Hubgeschwindigkeit während wenigstens eines der Hübe aus Einstechhub und Injektionshub von der Transportmutter beaufschlagbar. Hierdurch kann die Transportmutter neben ihrer Transportfunktion gleichzeitig auch zur exakten Steuerung der Hubbewegung während der eigentlichen Injektion dienen.

Ferner ist es günstig, wenn eine Steuerelektronik zur Steuerung der elektrischen Transporteinrichtung vorgesehen ist. Hierdurch ist es möglich, während der Hübe ein spezielles Geschwindigkeitsprofil vorzusehen beziehungsweise auch unterschiedliche Geschwindigkeitsprofile für verschiedene Injektionsanwendungen zu speichern.

In einer weiteren vorteilhaften Ausführungsform ist ein Einstechtiefenanschlag vorgesehen, an dem der Einstechschlitten bei Erreichen einer eingestellten Einstechtiefe anliegt. Gegen diesen wird der Einstechschlitten während des Injektionshubes, bei dem der Betätigungsstößel gegenüber dem übrigen Injektionsgerät in Injektionsrichtung bis in eine Endstellung verlagert wird, und einer daran anschließenden Verweilzeit gedrückt. Dabei ist der Betätigungsstößel und über ihn ein Stößel des Injektionsgerätes während der Verweilzeit in Injektionsrichtung in eine Endstellung vorgespannt und die Transportmutter von der Betätigungsvorrichtung beabstandet. Hierdurch wird sichergestellt, dass das Injektionsgerät während der Verweilzeit unter Vorspannung in seiner Endanschlagstellung gehalten wird. Die Verweilzeit dient dabei dazu, einerseits eine vollständige Entleerung des Injektionsflüssigkeitsbehälters zu gewährleisten und andererseits einen ausreichenden Abbau eines Überdruckes des injizierten Medikaments im Gewebe sicherzustellen. Dadurch wird vermieden, dass beim Herausziehen der Kanüle aus dem Gewebe, das Medikament dieser nachfolgt und dadurch in oberhalb gelegene Hautschichten gelangt.

Ferner ist es günstig, wenn ein Sensor zur Detektion der Endstellung vorgesehen ist, mittels dem ein einstellbares Zeitglied schaltbar ist, an dem die Dauer der Verweilzeit einstellbar ist. Hierdurch ist es möglich in Abhängigkeit der jeweils vorgesehenen Injektionsanwendungen unterschiedliche Verweilzeiten einzustellen.

Vorteilhafterweise ist der Einstechtiefenanschlag gegenüber dem Trägergehäuse verstellbar, um auch die Einstechtiefe an verschiedene Injektionsanwendungen anpassen zu können.

Zudem ist es günstig, wenn zwischen dem Einstechschlitten und dem Injektionsschlitten Verriegelungsmittel vorgesehen sind. Diese sind zwischen einer Verriegelungsstellung, in der ein Formschluss zwischen dem Einstechschlitten und dem Injektionsschlitten herstellbar ist, und einer Freigabestellung, in der der Einstechschlitten und der Injektionsschlitten relativ zueinander verschiebbar sind, verlagerbar. Dabei sind die Verriegelungsmittel mittels gehäuseseitiger Steuermittel in Abhängigkeit von der Position der Betätigungsvorrichtung selbsttätig zwischen der Freigabestellung und der Verriegelungsstellung verstellbar. Dies ermöglicht in zuverlässiger Weise ein selbsttätiges Umschalten der Injekionsvorrichtung zwischen dem Einstechhub und dem Injektionshub in Abhängigkeit ihrer Position gegenüber dem Trägergehäuse.

Vorteilhafterweise weisen dabei die Verriegelungsmittel eine am Injektionsschlitten drehgelagerte Wippe mit einem ersten Arm, der in der Verriegelungsstellung mit dem Einstechschlitten in Formschluss bringbar ist, und einen zweiten Arm auf, über den die Wippe gegenüber dem Trägergehäuse verschwenkbar ist. Hierdurch ist eine genaue positionsabhängige Verriegelung der beiden Schlitten mit einfachen Mitteln möglich.

Zudem ist es dabei günstig, wenn die Steuermittel ein erstes gehäuseseitiges Auslenkelement, insbesondere in Form einer ersten Rampe aufweisen, durch das der zweite Arm während des Einstechhubes bei Erreichen einer vorbestimmten Position des Einstechschlittens, die einer bestimmten Einstechtiefe des Injektionsgerätes entspricht, auslenkbar und dadurch die Wippe von der Verriegelungsstellung in die Freigabestellung verschwenkbar ist. Hierdurch ist ein exaktes Umschalten der Injektionsvorrichtung vom Einstechhub zum Injektionshub in Abhängigkeit vom Erreichen einer vorgegebenen Einstechtiefe möglich.

Ferner ist es günstig, wenn ein zweites gehäuseseitiges Auslenkelement, insbesondere in Form einer zweiten Rampe vorgesehen ist, durch das der zweite Arm während des Rückholhubes bei Erreichen einer Ausgangsstellung des Einstechschlittens auslenkbar und dadurch die Wippe von der Verriegelungsstellung in die Freigabestellung verschwenkbar ist, um den Einstechschlitten beim Rückholen der Betätigungsvorrichtung in einem geeigneten Zeitpunkt vom Injektionsschlitten entkoppeln zu können.

Hierbei ist es in jedem Fall vorteilhaft, wenn der zweite Arm gegen das Trägergehäuse vorgespannt ist, um eine störungsfreie Betätigung der Wippe durch wenigstens eines der gehäuseseitigen Auslenkelemente zu gewährleisten.

In einer alternativen vorteilhaften Ausführungsform weisen die Verriegelungsmittel einen in einer durchgehenden Ausnehmung des Einstechschlittens beweglich gehaltenen Kulissenstein auf, der in Abhängigkeit der Position des Einstechschlittens mittels einer als Steuermittel fungierenden gehäuseseitigen Kontur mit dem Injektionsschlitten in formschlüssiger Verbindung steht. Hierdurch können die Mittel zum Koppeln und Endkoppeln des Einstechschlittens und des Injektionsschlittens besonders kompakt ausgeführt werden.

Vorteilhafterweise wird der Einstechtiefenanschlag durch ein am Trägergehäuse verstellbar gelagertes Formteil gebildet, an dem auch die Auslenkmittel wenigstens teilweise ausgeformt sind. Auf diese Weise ist gewährleistet, dass beim Verstellen der Einstechtiefe auch die Position der Auslenkmittel automatisch angepasst wird.

Ferner ist es günstig, wenn das Formteil mittels einer von außerhalb des Trägergehäuses zugänglichen Stellschraube gegenüber diesem verschiebbar ist, wodurch die Einstechtiefe in einfacher Weise stufenlos verstellbar ist.

In den Figuren ist eine beispielhafte Ausführungsform der Erfindung dargestellt. Es zeigen:
- Figur 1: eine perspektivische Ansicht einer erfindungsgemäßen Injektionsvorrichtung,
- Figur 2: einen Längsschnitt durch die Injektionsvorrichtung nach Fig. 1,
- Figur 3a: eine vereinfachte geschnittene Darstellung der Injektionsvorrichtung nach Fig. 1 in einer Ausgangsstellung,
- Figur 3b: eine Darstellung der Injektionsvorrichtung nach Fig. 1 unmittelbar vor der Durchführung eines Mischhubes,
- Figur 3c: eine Darstellung der Injektionsvorrichtung nach Fig. 1 nach abgeschlossenem Mischhub,
- Figur 3d: eine Darstellung der Injektionsvorrichtung nach Fig. 1 zu Beginn eines Einstechhubes,
- Figur 3e: eine Darstellung der Injektionsvorrichtung nach Fig. 1 bei Übergang vom Einstechhub zu einem Injektionshub,
- Figur 3f: eine Darstellung der Injektionsvorrichtung nach Fig. 1 nach Abschluss des Injektionshubes beziehungsweise während einer Verweilzeit,
- Figur 3g: eine Darstellung der Injektionsvorrichtung nach Fig. 1 zu Beginn eines Rückholhubes,
- Figur 3h: eine Darstellung der Injektionsvorrichtung nach Fig. 1 während des Rückholhubes bei Erreichen einer Ausgangsstellung des Einstechschlittens,
- Figur 3i: eine Darstellung der Injektionsvorrichtung nach Fig. 1 während des Rückholhubes bei alleinigem Transport des Injektionsschlittens,
- Figur 4: eine Seitenansicht einer alternativen erfindungsgemäßen Injektionsvorrichtung,
- Figur 5: eine teilweise freigeschnittene Draufsicht auf die Injektionsvorrichtung in Richtung V aus Fig. 4,
- Figur 6: eine vergrößerte Darstellung des Details VI aus Fig. 5,
- Figur 7a: einen Schnitt durch die Betätigungsvorrichtung der Injektionsvorrichtung nach Fig. 4 in einer Ausgangsstellung,
- Figur 7b: einen Schnitt durch die Betätigungsvorrichtung nach Fig. 7a beim Umschalten zwischen Mischhub und Einstechhub,
- Figur 7c: einen Schnitt durch die Betätigungsvorrichtung nach Fig. 7a beim Umschalten zwischen Einstechhub Injektionshub,
- Figur 7d: einen Schnitt durch die Betätigungsvorrichtung nach Fig. 7a nach Abschluss des Injektionshubes beziehungsweise während der Verweilzeit und
- Figur 7e: einen Schnitt durch die Betätigungsvorrichtung nach Fig. 7a während des Rückholhubes.

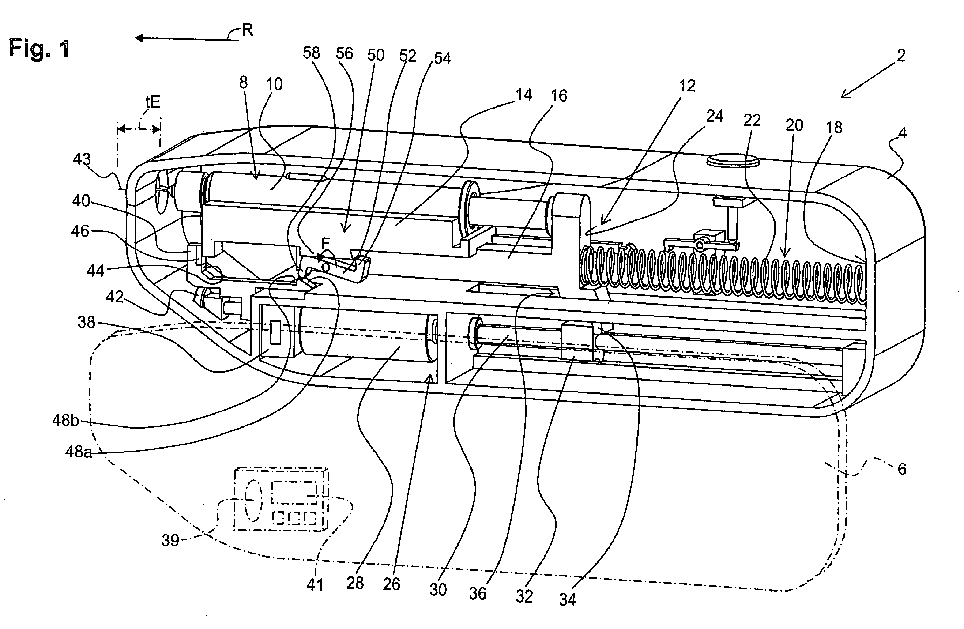

Fig. 1 zeigt eine erfindungsgemäße Injektionsvorrichtung 2 mit einem Trägergehäuse 4 bei entfernter Seitenwand 6. In die Injektionsvorrichtung 2 ist ein spritzenförmiges Injektionsgerät 8 mit einem Injektionsflüssigkeitsbehälter 10 eingesetzt. Dabei weist die Injektionsvorrichtung 2 zur Aufnahme und Verlagerung des Injektionsgerätes 8 entlang einer Injektionsrichtung R eine Betätigungsvorrichtung 12 auf, die sich im Wesentlichen aus einem Einstechschlitten 14, in dem das Injektionsgerät 8 gelagert ist, und einem Injektionsschlitten 16 zusammen setzt, über den das Injektionsgerät 8 auspressbar ist und der gegenüber dem Einstechschlitten 14 verlagerbar ist.

Zwischen dem Injektionsschlitten 16 und einer bezüglich der Injektionsrichtung R hinteren Rückwand 18 des Trägergehäuses 4 ist zur alleinigen Durckbeaufschlagung der Betätigungsvorrichtung 12 in Injektionsrichtung R eine Kraftspeichereinrichtung 20 vorgesehen. Diese ist im Wesentlichen durch ein Federmittel 22 gebildet, das den Injektionsschlitten 16 über eine Beaufschlagungsfläche 24 in Injektionsrichtung R vorspannt.

Ferner weist die Injektionsvorrichtung 2 eine elektrische Transporteinrichtung 26 auf, mittels der der Injektionsschlitten 16 beziehungsweise die Betätigungsvorrichtung 12 insgesamt ausschließlich entgegen der Injektionsrichtung R beaufschlagbar ist. Diese der Beaufschlagung durch die Kraftspeichereinrichtung 20 entgegen gesetzte Beaufschlagung durch die elektrische Transporteinrichtung 26 kann dabei sowohl zur Geschwindigkeitsbegrenzung in Injektionsrichtung R als auch zur Durchführung eines Rückholhubes aus einer Injektionsposition nach ausgeführter Injektionsanwendung zurück in eine Ausgangsposition der Betätigungsvorrichtung genutzt werden.

Die elektrische Transporteinrichtung 26 weist einen elektrischen Antriebsmotor 28 auf, mittels dem eine Gewindestange 30 rotatorisch antreibbar ist. Auf diese Gewindestange 30 ist eine Transportmutter 32 aufgeschraubt, die durch das Trägergehäuse 4 derart geführt ist, dass sie gegenüber diesem keine Drehbewegung vollziehen kann. Bei verdrehen der Gewindestange 30 durch den elektrischen Antriebsmotor 28 vollzieht die Transportmutter 32 vielmehr eine rein translatorische Bewegung parallel zur Injektionsrichtung R.

Zur Beaufschlagung der Betätigungsvorrichtung 12 durch die elektrische Transporteinrichtung 26 ist die Transportmutter 32 gegen einen Betätigungsanschlag 34 verfahrbar, der an einer der Injektionsrichtung R zugewandten Seite 36 des Injektionsschlittens 16 vorgesehen ist.

Die Drehrichtung und Drehzahl des elektrischen Antriebsmotors 28 und damit die Bewegungsrichtung und Geschwindigkeit der Transportmutter 32 kann dabei über eine Steuerelektronik 38 steuerbar sein, in der beispielsweise mindestens ein Geschwindigkeitsprofil für eine bestimmte Injektionsanwendung abgespeichert ist. Zudem kann die Steuerelektronik 38 mit Bedienungsmitteln 39 zur Steuerung des Antriebsmotors 28 oder mit Anzeigemitteln 41 beispielsweise zur Anzeige eines eingestellten Betriebsmodus, eines Lade- oder Betriebszustandes verbunden sein, wie durch strichpunktierte Linien dargestellt, Alternativ zu der dargestellten Anordnung an der Seitenwand 6 können die Bedienungsmittel 39 oder Anzeigemittel 41 in jeder gewünschten Position am Trägergehäuse 4 angeordnet sein. Zudem ist alternativ zu der Steuerung des Antriebsmotors 28 über eine Steuerelektronik 38 auch dessen direkte Steuerung über die Bedienungsmittel 39 oder eine vollautomatische Steuerung ohne jegliche Ansteuerungsmöglichkeit durch den Benutzer denkbar.

Ferner weist die Injektionsvorrichtung 2 ein Formteil 40 auf, das über eine von außerhalb des Trägergehäuses 4 betätigbare Stellschraube 42 innerhalb desselben translatorisch verlagerbar ist, um eine bestimmte Einstechtiefe tE festzulegen, die einer Länge entspricht, über die eine Kanüle 43 des Injektionsgerätes 8 aus dem Trägergehäuse 4 heraus verschiebbar ist. Hierzu bildet das Formteil 40 einen Einstechtiefenanschlag 44 aus, der den Bewegungspfad des Einstechschlittens 14 in Injektionsrichtung R begrenzt.

Zur optimierten Steuerung der Injektionsvorrichtung 2 kann dabei an dem Einstechtiefenanschlag 44 ein Sensor 46 zur Detektion einer Endstellung des Einstechschlittens 14 vorgesehen sein, der beispielsweise durch einen Mikroschalter gebildet und mit einem Zeitglied (nicht dargestellt) der Steuerelektronik 38 verbunden ist.

Einstückig mit dem Einstechtiefenanschlag 44 ist an dem Formteil 40 ein rampenförmiges Auslenkelement 48a ausgebildet. Ein weiteres rampenförmiges Auslenkelement 48b ist ferner direkt am Trägergehäuse 4 ausgeformt. Diese Auslenkelemente 48a, 48b fungieren als gehäuseseitige Steuermittel, mittels denen Verriegelungsmittel 50 der Betätigungsvorrichtung 12 betätigbar sind. Dabei können die Verriegelungsmittel 50, mittels denen eine formschlüssige Verbindung zwischen dem Einstechschlitten 14 und dem Injektionsschlitten 16 herstellbar ist, in Abhängigkeit der Position des Injektionsschlittens 16 gegenüber dem Formteil 40 beziehungsweise dem Trägergehäuse 4 von einer Verriegelungsstellung in eine Freigabestellung verbracht werden. Die Herstellung des Formschlusses zwischen beiden Schlitten 14, 16 ist dabei lediglich in der Verriegelungsstellung der Verriegelungsmittel 50 möglich.

Die Verriegelungsmittel 50 weisen hierzu eine am Injektionsschlitten 16 verschwenkbar gelagerte Wippe 52 auf. Diese bildet einen ersten Arm 54 aus, der an zwei Seiten eines Absatzes 56 des Einstechschlittens 14 anlegbar ist, um jeweils einen Formschluss herstellen zu können. Ferner weist die Wippe 52 einen zweiten Arm 58 auf, der mit den Auslenkelementen 48a, 48b zusammen wirkt. Zur Gewährleistung einer störungsfreien Betätigung der Wippe 52 durch die Auslenkelemente 48a, 48b ist diese dabei durch eine Federkraft F in die Verriegelungsstellung vorgespannt, in der der zweite Arm 58 gegen das Trägergehäuse 4 beziehungsweise gegen das daran gelagerte Formteil 40 drückt.

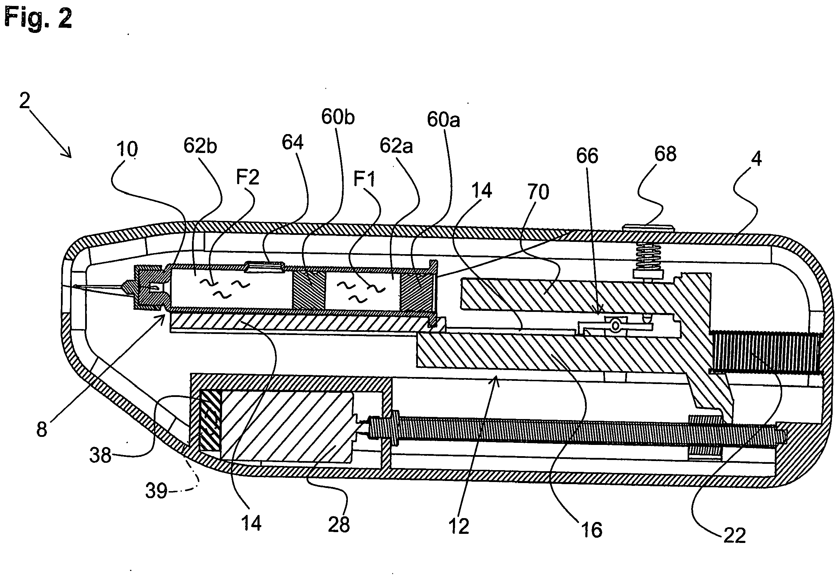

Fig. 2 zeigt einen Längsschnitt der Injektionsvorrichtung 2, der durch das Injektionsgerät 8 geht. Wie hieraus zu entnehmen ist, bildet der Injektionsflüssigkeitsbehälter 10 in der dargestellten Ausführungsform des Injektionsgerätes 8 mittels zweier Stopfen 60a, 60b zwei Kammern 62a, 62b aus, in denen ein Medium F1 und ein Medium F2 untergebracht sind. Die Medien F1, F2 dienen als zwei Komponenten eines zu injizierenden Medikamentes M, das erst unmittelbar vor dem Injektionsvorgang durch Vermischen beider Medien F1 und F2 bereitgestellt wird. Die Medien F1, F2 können hierbei gleiche oder unterschiedliche Aggregatzustände aufweisen. Dabei können beide Medien F1, F2, wie beispielhaft dargestellt, durch Flüssigkeiten gebildet sein. Alternativ hierzu ist es auch möglich, dass nur eines durch eine Flüssigkeit und das jeweils andere durch einen löslichen Feststoff, wie beispielsweise ein gefriergetrocknetes Pulver, gebildet ist. In jedem Fall können beide Medien M1, M2 eine unterschiedliche Wirkstoffzusammensetzung aufweisen, wobei es auch möglich ist, dass lediglich eines der Medien M1, M2 einen Wirkstoff enthält, während das jeweils andere lediglich zum Auflösen oder Verdünnen dient.

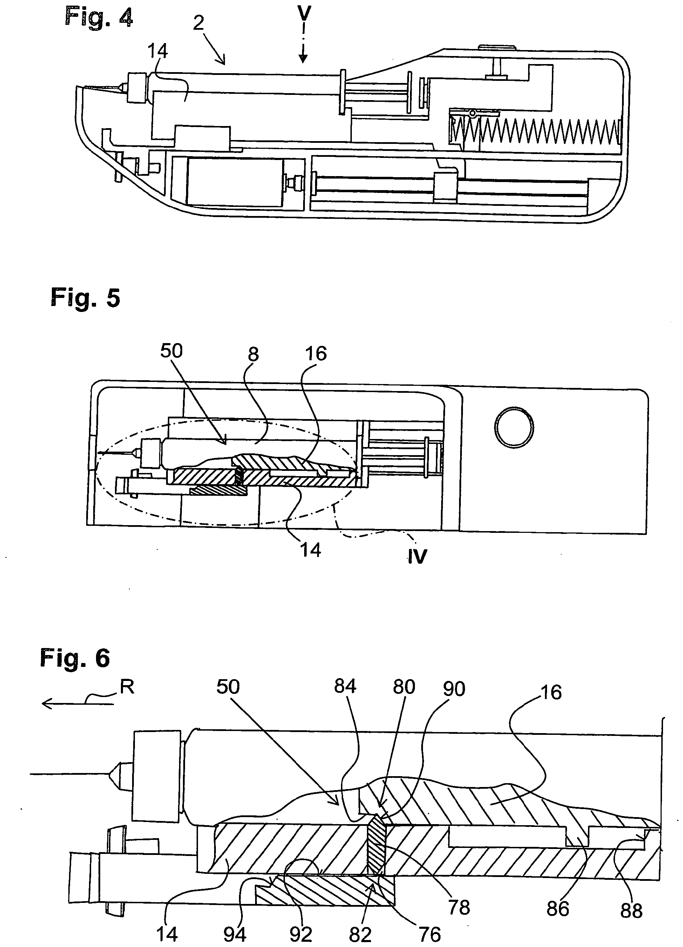

Der äußere Stopfen 60a fungiert bei dem gezeigten Injektionsgerät 8 gleichzeitig als ein von außen betätigbarer Spritzenstößel. Alternativ zum gezeigten Injektionsgerät 8 kann die Injektionsvorrichtung 2 selbstverständlich auch für Injektionsgeräte 8 in Form von Spritzen mit einer einzigen Kammer (nicht dargestellt) und/oder mit einem aus dem Injektionsflüssigkeitsbehälter 10 heraus ragenden Spritzenstößel (siehe Fig. 4) verwendet werden. Gegebenenfalls kann hierbei eine konstruktive Anpassung beziehungsweise Verstellbarkeit beispielsweise des Injektionsschlittens 8 notwendig sein.

Fig. 2 zeigt die Injektionsvorrichtung 2 in einer betriebsbereiten Ausgangsstellung. In dieser ist der Einstechschlitten 14 mittels einer Sicherungseinrichtung 66 in einer hinsichtlich der Injektionsrichtung R hinteren Position fixiert. Gleichzeitig wird in dieser Ausgangsstellung auch der Injektionsschlitten 16 durch die Transportmutter 32 entgegen der Kraft des Federmittels 22 in einer Position gehalten, in der ein zur Betätigung des Injektionsgerätes 8 vorgesehener Stößel 70 des Injektionsschlittens 16 von dem Injektionsgerät 8 beabstandet ist. Somit kann in dieser Ausgangsstellung der Injektionsvorrichtung 2 das Injektionsgerät 8 problemlos ein- oder ausgebaut werden.

Bei der Verwendung von Injektionsgerät-Typen entsprechend dem hier dargestellten Injektionsgerät 8, müssen die beiden Medien F1, F2 vor der Injektionsanwendung miteinander gemischt werden. Hierfür weist das Injektionsgerät 8 einen Umströmungskanal 64 auf, über den das Medium F1 um den zweiten Stopfen 60b herum dem Medium F2 zufließen kann, wenn dieser während eines so genannten Mischhubes der Betätigungsvorrichtung 12 auf die Höhe des Umströmungskanals 64 verschoben wird. Um einen solchen Mischhub bei Verwendung eines entsprechenden Injektionsgerätes 8 starten zu können, kann eine entsprechende Ansteuerung des Antriebsmotors 28 über die Bedienungsmittel 39 vorgesehen sein.

Der eigentliche Injektionsvorgang kann dann durch Betätigung eines von außerhalb des Trägergehäuses 4 zugänglichen Startknopfes 68 gestartet werden, durch den die Sicherungseinrichtung 66 gelöst wird.

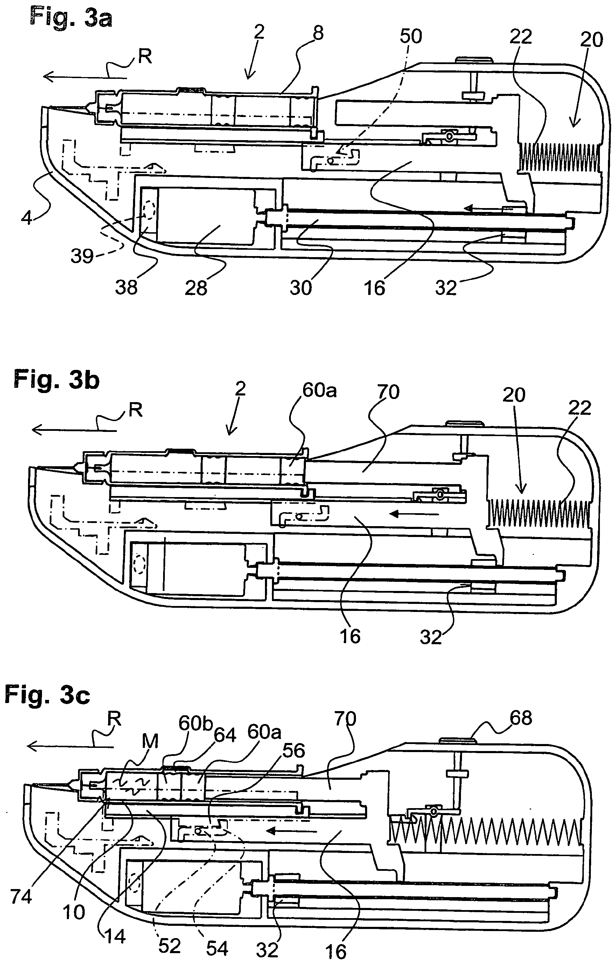

Die vereinfachten Schnittdarstellungen der Fig. 3a bis 3i stellen die prinzipielle Anordnung der wesentlichen Teile der Injektionsvorrichtung 2 während der Durchführung einer Injektionsanwendung dar. Zum besseren Verständnis wurden die Positionen der in dieser Schnittebene nicht sichtbaren Verriegelungsmittel 50 mit strichpunktierten Linien ergänzt.

Fig. 3a zeigt die Ausgangsstellung der Injektionsvorrichtung 2 entsprechend Fig. 2. In dieser ist das Injektionsgerät 8 vollständig innerhalb des Trägergehäuses 4 angeordnet und der Einstechschlitten 14 durch Eingriff der Sicherungseinrichtung 66 in seiner Position gesichert. Gleichzeitig ist das Federmittel 22 stark komprimiert durch den von der Transportmutter 32 bezüglich der Injektionsrichtung R relativ weit nach hinten verlagerten Injektionsschlitten 16.

Um bei Anwendung des dargestellten Injektionsgerätes 8 mit zwei zu mischenden Medien F1, F2 zunächst den Mischhub durchzuführen, wird der Antriebsmotor 28 über die Bedienungsmittel 39 gestartet. Hierbei wird die Gewindestange 30 derart verdreht, dass die Transportmutter 32 in Injektionsrichtung R verlagert wird. Da der Injektionsschlitten 16 zwischen der Transportmutter 32 und der Kraftspeichereinrichtung 20 eingespannt ist, wird dieser hierbei durch die Kraft des Federmittels 22 ebenfalls in Injektionsrichtung R verschoben, so dass der Stößel 70 in Anlage mit dem ersten Stopfen 60a kommt, wie in Fig. 3b dargestellt.

Die Transportmutter 32 wird dann, wie in Fig. 3c dargestellt, im Weiteren bis in eine injektionsrichtungsseitige Endstellung verlagert, während der Injektionsschlitten 16 allein unter Druckbeaufschlagung der Kraftspeichereinrichtung 20 weiter in Injektionsrichtung R verschoben wird. Dabei verlagert der Betätigungsstößel 70 den ersten Stopfen 60a wie auch den zweiten Stopfen 60b derart, dass sich die Medien F1, F2 zunächst über den Umströmungskanal 64 miteinander vermischen können (nicht dargestellt) und dadurch ein für die Injektion vorgesehenes Medikament M bilden. Anschließend steht dieses Medikament, wie dargestellt, an einem kanülenseitigen Ende 74 des Injektionsflüssigkeitsbehälters 10 zur Verfügung. In dieser Position ist der Mischhub der Injektionsvorrichtung 2 abgeschlossen.

Gleichzeitig kommt der erste Arm 54 der Wippe 52 in Eingriff mit dem Absatz 56, so dass über die Wippe 52 ein in Injektionsrichtung R wirkender Formschluss zwischen dem Einstechschlitten 14 und dem Injektionsschlitten 16 hergestellt ist. Da der Einstechschlitten 14 nach wie vor über die Sicherungseinrichtung 66 in seiner Position festgelegt ist, wird über den Formschluss nun auch der Injektionsschlitten 16 in dieser Stellung fixiert.

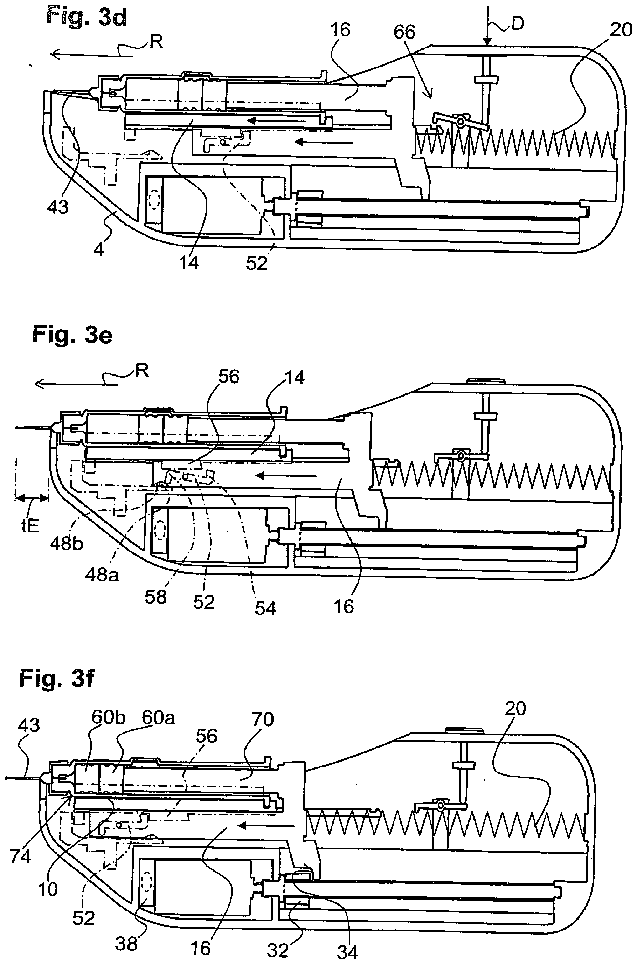

Durch Aufbringung einer Druckkraft D auf den Startknopf 68 kann nun aus dieser Position heraus der eigentliche Injektionsvorgang mit dem Einstechhub gestartet werden. Hierbei wird die Sicherungseinrichtung 66 wie in Fig. 3d dargestellt gelöst und der Injektionsschlitten 16 mittels der Druckbeaufschlagung durch die Kraftspeichereinrichtung 20 weiter in Injektionsrichtung R verlagert. Durch den Formschluss über die Wippe 52 wird hierbei auch der Einstechschlitten 14 mit bewegt, so dass nun der Einstechhub gestartet wird, bei dem die Kanüle 43 aus dem Trägergehäuse 4 austritt.

Sobald der Einstechschlitten 14 dabei, wie in Fig. 3e dargestellt, durch Anlage an den Einstechtiefenanschlag 44 die eingestellte Einstechtiefe tE erreicht hat, wird der zweite Arm 58 der Wippe 52 durch das Auslenkelement 48a nach oben gedrückt und dadurch die Wippe 52 aus ihrer Verriegelungsstellung in die Freigabestellung verschwenkt, in der der erste Arm 54 bei der weiteren Bewegung des Injektionsschlittens 16 in Injektionsrichtung R an dem Absatz 56 vorbei bewegt werden kann. Hierdurch wird der Einstechhub beendet und der Injektionshub gestartet.

Bei der Weiterbewegung des Injektionsschlittens 16 mittels der Kraftspeichereinrichtung 20 verschiebt nun der Betätigungsstößel 70 die beiden Stopfen 60a, 60b in Injektionsrichtung R und presst dadurch das Medikament über die Kanüle 43 aus bis der bezüglich der Injektionsrichtung R vordere Stopfen 60b am kanülenseitigen Ende 74 des Injektionsflüssigkeitsbehälters 10 anschlägt, wie in Fig. 3f dargestellt, und der Injektionshub somit abgeschlossen ist.

Die Kraftspeichereinrichtung 20 beaufschlagt den Injektionsschlitten 16 in dieser Position weiter mit einer Druckkraft, während die Transportmutter 32 in ihrer Endstellung verbleibt, in der sie zum Betätigungsanschlag 34 beabstandet ist. Hierdurch bleiben die beiden Stopfen 60a, 60b während einer über die Steuerelektronik 38 vorbestimmten Verweilzeit gegen das kanülenseitige Ende 74 vorgespannt. Auf diese Weise wird gewährleistet, dass sich der Injektionsflüssigkeitsbehälter 10 vollständig entleert und sich ein durch das Medikament M entstehender Überdruck im injizierten Gewebe (nicht dargestellt) abbauen kann.

Ferner hat die Wippe 52 in dieser Position den Absatz 56 vollständig passiert, so dass diese durch die Federkraft F wieder in ihre Verriegelungsstellung zurück verschwenkt wird.

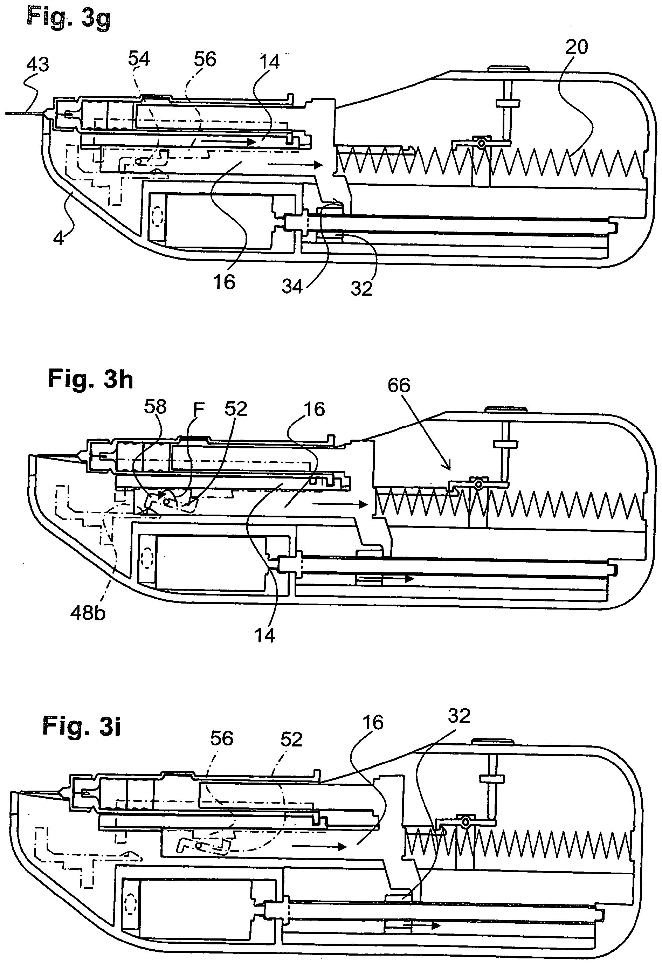

Nach Ablauf der Verweilzeit setzt die Steuerelektronik 38 den Antriebsmotor 28 nun in entgegen gesetzter Richtung als zuvor in Betrieb, wodurch die Transportmutter 32 entgegen der Injektionsrichtung R verlagert wird und dadurch der Rückholhub gestartet wird. Hierbei wird die Transportmutter 32 an den Betätigungsanschlag 34 angelegt, wie in Fig. 3g dargestellt, und drückt den Injektionsschlitten 16 entgegen der Druckkraft der Kraftspeichereinrichtung 20 zurück in Richtung der Ausgangsstellung.

Gleichzeitig drückt der erste Arm 54 nun von der anderen Seite gegen den Absatz 56 und verschiebt über diesen auch den Einstechschlitten 14, so dass die Kanüle 43 wieder in das Trägergehäuse 4 hinein verschoben wird. Sobald der Einstechschlitten 14 dabei wieder seine Ausgangsstellung erreicht hat, wird der zweite Arm 58 der Wippe 52 nun von dem Auslenkelement 48b entgegen der Federkraft F verschwenkt und dadurch die Verriegelung mit dem Injektionsschlitten 16 erneut aufgehoben. Gleichzeitig wird in dieser Position der Einstechschlitten 14 wieder über die Sicherungseinrichtung 66 fixiert, wie in Fig. 3h dargestellt.

Daraufhin wird der Injektionsschlitten 16 durch die Transportmutter 32 weiter in Richtung seiner Ausgangsposition verlagert, wobei die Wippe 52 wieder in die Verriegelungsstellung zurück schwenkt, sobald sie den Absatz 56 vollständig passiert hat, wie in Fig. 3i dargestellt.

Sobald die Ausgangsstellung gemäß Fig. 3a wieder erreicht ist, kann dann das Injektionsgerät 8 entnommen und ein anderes eingesetzt werden, um den nächsten Injektionsvorgang starten zu können.

Alternativ zu der Ausführungsform der Injektionsvorrichtung 2 entsprechend Fig. 1 bis 3 ist es auch möglich an Stelle der Wippe 52, des Absatzes 56 und der Auslenkelemente 48a, 48b andere Verriegelungsmittel zwischen dem Einstechschlitten 14 und dem Injektionsmittel 16 beziehungsweise andere Steuermittel zu deren positionsabhängigen Betätigung vorzusehen.

Die Fig. 4 bis 6 zeigen beispielhaft eine Ausführungsform der Injektionsvorrichtung 2 mit alternativ verwendbaren Verriegelungsmitteln 50. Hierbei ist in den Einstechschlitten 14 eine senkrecht zur Injektionsrichtung R angeordnete Durchgangsbohrung 76 eingelassen (siehe Fig. 6), in der ein Kulissenstein 78 beweglich gelagert ist. Der Kulissenstein 78 weist ein injektionsschlittenseitiges Ende 80 und ein formteilseitiges Ende 82 auf. Dabei ist der Kulissenstein 78 derart dimensioniert, dass er wenigstens mit einem der beiden Enden 80, 82 aus der Durchgangsbohrung 76 heraus ragt.

Entsprechend der Verriegelungsfunktion der Wippe 52 gemäß Fig. 3a bis 3i steht der Einstechschlitten 14 auch bei dieser Ausführungsform während des Einstechhubes und während des Rückholhubes in formschlüssiger Verbindung mit dem Injektionsschlitten 16. Der Injektionsschlitten 16 weist hierzu eine Eingriffsausnehmung 84, mit der das injektionsschlittenseitige Ende 80 des Kulissensteins 78 in Injektionsrichtung R in Eingriff bringbar ist, sowie einen vom übrigen Injektionsschlitten 16 abstehenden Mitnehmer 86 auf, der entgegen der Injektionsrichtung R an einen in den Einstechschlitten 14 eingelassenen Absatz 88 anlegbar ist.

Zur Verlagerung des Kulissensteins 78 zwischen seiner Verriegelungsposition und seiner Freigabeposition hinsichtlich des Injektionsschlittens 16 weist dieser an seinem injektionsschlittenseitigen Ende 80 eine erste schräge Anschlagsfläche 90 auf, über die der Kulissenstein 78 durch Anpressdruck der Eingriffsausnehmung 84 gegen das Formteil 40 vorgespannt wird.

Zur positionsabhängigen Anordnung des Kulissensteins 78 entlang der Durchgangsbohrung 76 weist das Formteil 40 in dieser Ausführungsform auf Höhe der Durchgangsbohrung 76 eine am Einstechschlitten 14 anliegende Steuerwand 92 auf. Am Umschaltpunkt zwischen Einstechhub und Injektionshub schließt sich an diese Steuerwand 92 eine schräge Steuerfläche 94 an, die sich vom Einstechschlitten 14 weg erstreckt.

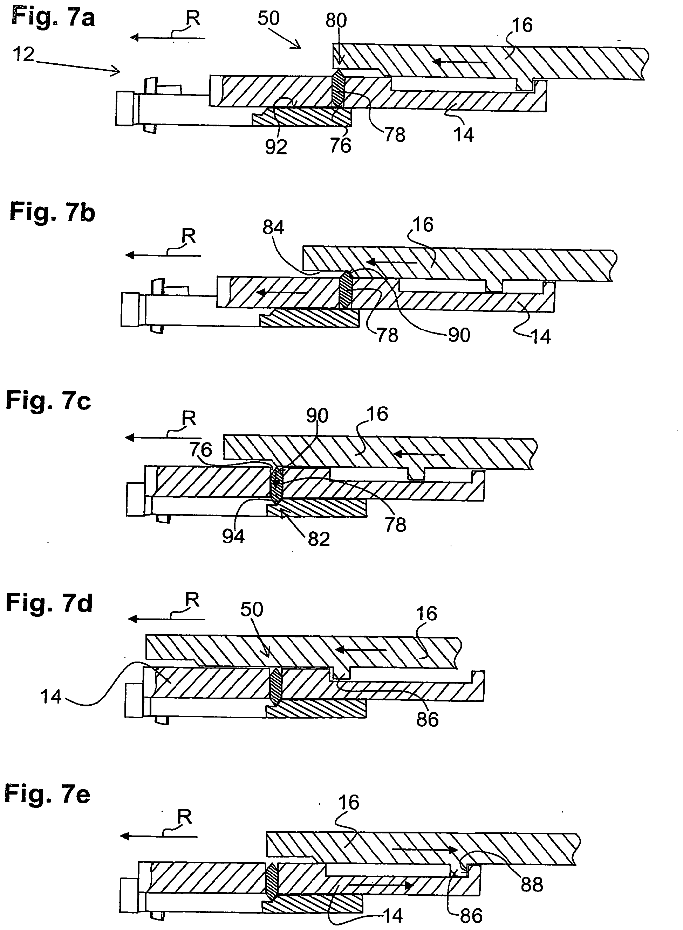

Der Ablauf eines Injektionsvorganges dieser Injektionsvorrichtung 2 umfasst entsprechend dem Ablauf nach Fig.3a bis 3i einen optionalen Mischhub, einen Einstechhub, einen Injektionshub und einen Rückholhub und läuft entsprechend der Darstellung in den Fig. 7a bis 7e ab. Die übrige Funktionsweise und insbesondere die Beaufschlagung der Betätigungsvorrichtung 12 durch die Kraftspeichereinrichtung 20 und die elektrische Transporteinrichtung entspricht dabei der Funktionsweise gemäß Fig. 3a bis 3i.

Fig. 7a zeigt die Verriegelungsmittel 50 nach Fig. 6 in der Ausgangsstellung. Hierbei steht das injektionsschlittenseitige Ende 80 des Kulissensteins 78 in Folge der direkt an der Durchgangsbohrung 76 anliegenden Steuerwand 92 aus der Durchgangsbohrung 76 heraus. Der Injektionsschlitten 16 ist jedoch beabstandet zum Kulissenstein 78 angeordnet und kann somit während eines Mischhubes in Injektionsrichtung R unabhängig vom Einstechschlitten 14 verschoben werden.

Bei Übergang vom Mischhub zum Einstechhub kommt der Injektionsschlitten 16 an der Eingriffsausnehmung 84 in Anlage an die Anschlagsfläche 90 des Kulissensteins 78. Bei der weiteren Bewegung des Injektionsschlittens 16 in Injektionsrichtung R wird somit der Einstechschlitten 14 über den Kulissenstein 78 mit bewegt.

Sobald der Kulissenstein 78 hierbei auf die Höhe der Steuerfläche 94 verschoben ist, wird er in Folge des Druckes des Injektionsschlittens 16 auf die schräge Anschlagsfläche 90 in die Durchgangsbohrung 76 hinein verschoben bis er lediglich noch mit dem formteilseitigen Ende 82 aus dieser heraus ragt (siehe Fig. 7c) Hierdurch wird der Einstechhub abgeschlossen und der Injektionshub gestartet.

Fig. 7d zeigt die Verriegelungsmittel 50 bei Abschluss des Injektionshubes beziehungsweise während der Verweilzeit. Um in dieser Position entsprechend der Ausführungsform nach Fig. 1 bis 3 eine Vorspannung des Injektionsgerätes 8 (nicht dargestellt) in die Endstellung zu ermöglichen, liegt der Injektionsschlitten 16 in Injektionsrichtung R nicht am Einstechschlitten 14 an, sondern ist, insbesondere am Mitnehmer 86, zu diesem beabstandet.

Bei Durchführung des Rückholhubes kommt bei dem entgegen der Injektionsrichtung erfolgenden Transport des Injektionsschlittens 16 der Mitnehmer 86 in Anlage an den Absatz 88, wie in Fig. 7e dargestellt, so dass der Einstechschlitten 14 zusammen mit diesem wieder in die Ausgangsstellung zurück transportiert werden kann.

## Patentansprüche

1. Injektionsvorrichtung (2)
mit einem Trägergehäuse (4), in dem ein Injektionsgerät (8) mit wenigstens einem auspressbaren Injektionsflüssigkeitsbehälter (10) einsetzbar ist,
und einer zur Aktivierung des Injektionsgerätes (8) entlang einer Injektionsrichtung (R) antreibbaren Betätigungsvorrichtung (12) zur Aufbringung von Antriebskräften auf das Injektionsgerät (8), die von einer mechanischen Kraftspeichereinrichtung (20) und einer elektrischen Transporteinrichtung (26) beaufschlagbar ist,
**dadurch gekennzeichnet, dass** die Betätigungsvorrichtung (12) zur Ausführung wenigstens eines Einstechhubes und eines Injektionshubes allein durch die Antriebskraft einer mechanischen Kraftspeichereinrichtung (20) angetrieben ist und die elektrische Transporteinrichtung (26) eine Geschwindigkeitsbegrenzung bildet, die während einer Injektion zur Begrenzung einer durch die Kraftspeichereinrichtung erzielten Hubgeschwindigkeit entgegengesetzt zur Antriebskraft wirkt.

2. Injektionsvorrichtung nach Anspruch 1, **dadurch gekennzeichnet, dass** die Kraftspeichereinrichtung (20) ein zwischen der Betätigungsvorrichtung (12) und dem Trägergehäuse (4) eingespanntes Federmittel (22) aufweist.

3. Injektionsvorrichtung nach Anspruch 1 oder 2, **dadurch gekennzeichnet, dass** die Betätigungsvorrichtung (12) von der elektrischen Transporteinrichtung (26) lediglich entgegen der Injektionsrichtung (R) beaufschlagbar ist.

4. Injektionsvorrichtung nach Anspruch 3, **dadurch gekennzeichnet, dass** die Betätigungsvorrichtung (12) durch die elektrische Transporteinrichtung (26) entgegen der Injektionsrichtung (R) von einer Injektionsposition in eine Ausgangsposition verbringbar ist.

5. Injektionsvorrichtung nach einem der Ansprüche 1 bis 4, **dadurch gekennzeichnet, dass** die Betätigungsvorrichtung (12) einen Einstechschlitten (14) mit einer Aufnahme für das Injektionsgerät (8) sowie einen relativ zum Einstechschlitten (14) verlagerbaren Injektionsschlitten (16) aufweist, der einen Betätigungsstößel (70) für die Beaufschlagung eines Stößels des Injektionsgerätes (8) aufweist, wobei der Einstechschlitten (14) und der Injektionsschlitten (16) von der mechanischen Kraftspeichereinrichtung (20) und der elektrischen Transporteinrichtung (26) wenigstens zur Durchführung eines Einstechhubes, eines Injektionshubes und eines Rückholhubes ansteuerbar sind.

6. Injektionsvorrichtung nach Anspruch 5, **dadurch gekennzeichnet, dass** der Injektionsschlitten (16) von der mechanischen Kraftspeichereinrichtung (20) und der elektrischen Transporteinrichtung (26) zusätzlich zur Durchführung eines vorgeschalteten Mischhubes ansteuerbar ist.

7. Injektionsvorrichtung nach Anspruch 5 oder 6, **dadurch gekennzeichnet, dass** die elektrische Transporteinrichtung (26) eine Transportmutter (32) aufweist, die zum Transport der Betätigungsvorrichtung (12) während des Rückholhubes gegen einen Betätigungsanschlag (34) an einer der Injektionsrichtung (R) zugewandten Seite (36) der Betätigungsvorrichtung (12) anlegbar ist.

8. Injektionsvorrichtung nach Anspruch 7, **dadurch gekennzeichnet, dass** die Betätigungsvorrichtung (12) zur Steuerung einer Hubgeschwindigkeit während wenigstens eines der Hübe aus Mischhub, Einstechhub und Injektionshub von der Transportmutter (32) beaufschlagbar ist.

9. Injektionsvorrichtung nach einem der Ansprüche 1 bis 8, **dadurch gekennzeichnet, dass** eine Steuerelektronik (38) zur Steuerung der elektrischen Transporteinrichtung (26) vorgesehen ist.

10. Injektionsvorrichtung nach einem der Ansprüche 5 bis 9, **dadurch gekennzeichnet, dass** ein Einstechtiefenanschlag (44) vorgesehen ist, an dem der Einstechschlitten (14) bei Erreichen einer eingestellten Einstechtiefe (tE) anliegt und gegen den der Einstechschlitten (14) während des Injektionshubes, bei dem der Betätigungsstößel (70) gegenüber dem übrigen Injektionsgerät (8) in Injektionsrichtung (R) bis in eine Endstellung verlagert wird, und einer daran anschließenden Verweilzeit gedrückt ist,
wobei der Betätigungsstößel (70) während der Verweilzeit in Injektionsrichtung (R) in eine Endstellung vorgespannt und die Transportmutter (32) von der Betätigungsvorrichtung (12) beabstandet ist.

11. Injektionsvorrichtung nach Anspruch 10, **dadurch gekennzeichnet, dass** ein Sensor (46) zur Detektion der Endstellung vorgesehen ist, mittels dem ein einstellbares Zeitglied schaltbar ist, an dem die Dauer der Verweilzeit einstellbar ist.

12. Injektionsvorrichtung nach Anspruch 10 oder 11, **dadurch gekennzeichnet, dass** der Einstechtiefenanschlag (44) gegenüber dem Trägergehäuse (4) verstellbar ist.

13. Injektionsvorrichtung nach einem der Ansprüche 5 bis 12, **dadurch gekennzeichnet, dass** zwischen dem Einstechschlitten (14) und dem Injektionsschlitten (16) Verriegelungsmittel (50) vorgesehen sind, die zwischen einer Verriegelungsstellung, in der ein Formschluss zwischen dem Einstechschlitten (14) und dem Injektionsschlitten (16) herstellbar ist, und einer Freigabestellung, in der der Einstechschlitten (14) und der Injektionsschlitten (16) relativ zueinander verschiebbar sind, verlagerbar sind, wobei die Verriegelungsmittel (50) mittels gehäuseseitiger Steuermittel in Abhängigkeit von der Position der Betätigungsvorrichtung (12) selbsttätig zwischen Freigabestellung und Verriegelungsstellung verstellbar sind.

14. Injektionsvorrichtung nach Anspruch 13, **dadurch gekennzeichnet, dass** die Verriegelungsmittel (50) eine am Injektionsschlitten (16) drehgelagerte Wippe (52) mit einem ersten Arm (54), der in der Verriegelungsstellung mit dem Einstechschlitten (14) in Formschluss bringbar ist, und einem zweiten Arm (58), über den die Wippe (52) gegenüber dem Trägergehäuse (4) verschwenkbar ist, aufweist.

15. Injektionsvorrichtung nach Anspruch 14, **dadurch gekennzeichnet, dass** die Steuermittel ein erstes gehäuseseitiges Auslenkelement (48a) aufweisen, durch das der zweite Arm (58) während des Einstechhubes bei Erreichen einer vorbestimmten Position des Einstechschlittens (14) auslenkbar und dadurch die Wippe (52) von der Verriegelungsstellung in die Freigabestellung verschwenkbar ist.

16. Injektionsvorrichtung nach Anspruch 14 oder 15, **dadurch gekennzeichnet, dass** ein zweites gehäuseseitiges Auslenkelement (48b) vorgesehen ist, durch das der zweite Arm (58) während des Rückholhubes bei Erreichen einer Ausgangsstellung des Einstechschlittens (14) auslenkbar und dadurch die Wippe (52) von der Verriegelungsstellung in die Freigabestellung verschwenkbar ist.

17. Injektionsvorrichtung nach Anspruch 15 oder 16, **dadurch gekennzeichnet, dass** der zweite Arm (58) gegen das Trägergehäuse (4) vorgespannt ist.

18. Injektionsvorrichtung nach Anspruch 13, **dadurch gekennzeichnet, dass** die Verriegelungsmittel (50) einen in einer durchgehenden Ausnehmung des Einstechschlittens (14) beweglich gehaltenen Kulissenstein (78) aufweisen, der in Abhängigkeit der Position des Einstechschlittens (14) mittels einer als Steuermittel fungierenden gehäuseseitigen Kontur mit dem Injektionsschlitten (16) in formschlüssiger Verbindung steht.

19. Injektionsvorrichtung nach einem der Ansprüche 13 bis 18, **dadurch gekennzeichnet, dass** der Einstechtiefenanschlag (44) durch ein am Trägergehäuse (4) verstellbar gelagertes Formteil (40) gebildet ist, an dem auch die Auslenkelemente (48a, 48b) wenigstens teilweise ausgeformt sind.

20. Injektionsvorrichtung nach Anspruch 19, **dadurch gekennzeichnet, dass** das Formteil (40) mittels einer von außerhalb des Trägergehäuses (4) zugänglichen Stellschraube (42) gegenüber diesem verschiebbar ist.

## Claims

1. Injection apparatus (2)
comprising a carrier housing (4), into which an injection device (8), having at least one injection fluid container (10) that can be pressed, can be inserted,
and an actuating apparatus (12) which can be driven along an injection direction (R) in order to activate the injection device (8) in order to apply drive forces onto the injection device (8), which can be acted on by a mechanical energy storage unit (20) and an electrical transport unit (26),
**characterised in that** the actuating apparatus (12), in order to carry out at least one insertion stroke and one injection stroke, is driven solely by the drive force of a mechanical energy storage unit (20), and the electrical transport unit (26) forms a speed limiter, which, during an injection, takes effect counter to the drive force in order to limit the stroke speed attained by the energy storage unit.

2. Injection apparatus according to claim 1, **characterised in that** the energy storage unit (20) comprises a spring means (22) tensioned between the actuating apparatus (12) and the carrier housing (4).

3. Injection apparatus according to claims 1 and 2, **characterised in that** the actuating apparatus (12) can be subjected to force by the electrical transport unit (26) only counter to the injection direction (R).

4. Injection apparatus according to claim 3, **characterised in that** the actuating apparatus (12) can be brought by the electrical transport unit (26) against the injection direction (R) from an injection position into an initial position.

5. Injection apparatus according to any one of claims 1 to 4, **characterised in that** the actuating apparatus (12) comprises an insertion carriage (14) with a mounting for the injection device (8), as well as an injection carriage (16) which can be displaced relative to the insertion carriage (14), which comprises an actuation plunger (70) for imposing force onto a ram of the injection device (8), wherein the insertion carriage (14) and the injection carriage (16) can be actuated by the mechanical energy storage device (20) and the electrical transport unit (26) at least for carrying out an insertion stroke, an injection stroke, and a return stroke.

6. Injection apparatus according to claim 5, **characterised in that** the injection carriage (16) can be actuated by the mechanical energy storage device (20) and the electrical transport unit (26) additionally in order to carry out a preliminary mixing stroke.

7. Injection apparatus according to claim 5 or 6, **characterised in that** the electrical transport (26) comprises a transport nut (32), which, for transporting the actuating apparatus (12) during the return stroke, can be brought in contact against an actuation stop (34) on a side (36) of the actuating apparatus (12) facing towards the injection direction (R).

8. Injection apparatus according to claim 7, **characterised in that,** in order to control a stroke speed during at least one of the strokes from among the mixing stroke, insertion stroke, and injection stroke, the actuating apparatus (12) can be subjected to force by the transport nut (32).

9. Injection apparatus according to any one of claims 1 to 8, **characterised in that** a control electronics unit (38) is provided for controlling the electrical transport unit (26).

10. Injection apparatus according to any one of claims 5 to 9, **characterised in that** an insertion depth stop (44) is provided, at which the insertion carriage (14) comes in contact on reaching a predetermined insertion depth (tE) and against which the insertion carriage (14) is pressed during the injection stroke, during which the actuating plunger (70) is displaced in relation to the other elements of the injection device (8) in the injection direction (R) as far as into an end position, and during a dwell time period following this,
wherein the actuating plunger (70) is pre-tensioned during the dwell time in the injection direction (R) into an end position, and the transport nut (32) is located at a distance from the actuating apparatus (12).

11. Injection apparatus according to claim 10, **characterised in that** a sensor (46) is provided for detecting the end position, by means of which an adjustable timer element can be switched, by which the duration of the dwell time can be adjusted.

12. Injection apparatus according to claim 10 or 11, **characterised in that** the insertion depth stop (44) can be adjusted in relation to the carrier housing (4).

13. Injection apparatus according to any one of claims 5 to 12, **characterised in that** locking means (50) are provided between the insertion carriage (14) and the injection carriage (16), which can be moved between a locking position, in which a positive fit connection can be established between the insertion carriage (14) and the injection carriage (16), and a release position in which the insertion carriage (14) and the injection carriage (16) can be displaced relative to one another, wherein the locking means (50) can be adjusted automatically in position by means of housing-side control means, as a dependency of the position of the actuating apparatus (12), between the release position and the locking position.

14. Injection apparatus according to claim 13, **characterised in that** the locking means (50) comprise a rocker switch (52), rotatably mounted on the injection carriage (16), with a first arm (54) which in the locking position can be brought into positive fit connection with the insertion carriage (14), and a second arm (58) by means of which the rocker switch (52) can be pivoted in relation to the carrier housing (4).

15. Injection apparatus according to claim 14, **characterised in that** the control means comprise a first housing-side deflection element (48a), by means of which the second arm (58), during the insertion stroke, can be deflected on reaching a predetermined position of the insertion carriage (14), and, as a result, the rocker switch (52) can be pivoted from the locking position into the release position.

16. Injection apparatus according to any one of claims 14 or 15, **characterised in that** a second housing-side deflection element (48b) is provided, by means of which the second arm (58), during the return stroke, can be deflected on reaching an initial position of the insertion carriage (14), and thereby the rocker switch (52) can be pivoted from the locking position into the release position.

17. Injection apparatus according to any one of claims 15 or 16, **characterised in that** the second arm (58) is pre-tensioned against the carrier housing (4).

18. Injection apparatus according to claim 13, **characterised in that** the locking means (50) comprise a slide block (78), which is movably held in a continuous cut-out opening of the insertion carriage (14), which, as a dependency of the position of the insertion carriage (14), by means of a housing-side contour functioning as control means, is in positive fit connection with the injection carriage (16).

19. Injection apparatus according to any one of claims 13 to 18, **characterised in that** the insertion depth stop (44) is formed by a moulded part (40) adjustably mounted at the carrier housing (4), at which the deflection elements (48a, 48b) are also at least partially formed.

20. Injection apparatus according to claim 19, **characterised in that** the moulded part (40) can be moved in relation to the carrier housing (4) by means of a setting screw (42) accessible from outside the carrier housing.

## Revendications

1. Dispositif d'injection (2)
comprenant un boitier support (4), dans lequel peut être placé un appareil d'injection (8) comprenant au moins un réservoir de liquide d'injection (10) pouvant être pressé,
et un dispositif d'actionnement (12) pour l'application de forces d'entraînement sur l'appareil d'injection (8), le dispositif d'actionnement pouvant être entraîné dans une direction d'injection (R) pour l'activation de l'appareil d'injection (8), et pouvant être soumis à l'action d'un dispositif mécanique accumulateur de force (20) et d'un dispositif électrique de transport (26),
**caractérisé en ce que** le dispositif d'actionnement (12) est entraîné seulement par la force d'entraînement d'un dispositif mécanique accumulateur de force (20) pour réaliser au moins une course de piqûre et une course d'injection et le dispositif électrique de transport (26) forme un limiteur de vitesse, qui pendant une injection agit de manière opposée à la force d'entraînement pour limiter une vitesse de course atteinte du fait du dispositif accumulateur de force.

2. Dispositif d'injection selon la revendication 1, **caractérisé en ce que** le dispositif accumulateur de force (20) comprend un moyen de ressort (22) fixé entre le dispositif d'actionnement (12) et le boîtier support (4).

3. Dispositif d'injection selon la revendication 1 ou 2, **caractérisé en ce que** le dispositif d'actionnement (12) peut être soumis à l'action du dispositif électrique de transport (26) seulement à l'encontre de la direction d'injection (R).

4. Dispositif d'injection selon la revendication 3, **caractérisé en ce que** le dispositif d'actionnement (12) peut être passé depuis une position d'injection à une position de sortie par le dispositif électrique de transport (26) à l'encontre de la direction d'injection (R).

5. Dispositif d'injection selon l'une des revendications 1 à 4, **caractérisé en ce que** le dispositif d'actionnement (12) comprend un chariot de piqûre (14) comprenant un logement pour l'appareil d'injection (8) ainsi qu'un chariot d'injection (16) pouvant être déplacé relativement par rapport au chariot de piqûre (14), ledit chariot d'injection comprenant un poussoir d'actionnement (70) pour la sollicitation d'un poussoir de l'appareil d'injection (8), le chariot de piqûre (14) et le chariot d'injection (16) pouvant être commandés au moins pour réaliser une course de piqûre, une course d'injection et une course de retrait par le dispositif mécanique accumulateur de force (20) et le dispositif électrique de transport (26).

6. Dispositif d'injection selon la revendication 5, **caractérisé en ce que** le chariot d'injection (16) peut être commandé en outre pour réaliser une course de mélange en amont par le dispositif mécanique accumulateur de force (20) et le dispositif électrique de transport (26).

7. Dispositif d'injection selon la revendication 5 ou 6, **caractérisé en ce que** le dispositif électrique de transport (26) comprend un écrou de transport (32) qui peut être appliqué sur un côté (36) du dispositif d'actionnement (12) opposé à la direction d'injection (R) contre une butée d'actionnement (34) pour le transport du dispositif d'actionnement (12) pendant la course de retrait.

8. Dispositif d'injection selon la revendication 7, **caractérisé en ce que** le dispositif d'actionnement (12) peut être soumis à l'action de l'écrou de transport (32) pour la commande d'une vitesse de course pendant au moins une des courses parmi une course de mélange, une course de piqûre et une course d'injection.

9. Dispositif d'injection selon l'une des revendications 1 à 8, **caractérisé en ce qu'**il est prévu une électronique de commande (38) pour la commande du dispositif électrique de transport (26).

10. Dispositif d'injection selon l'une des revendications 5 à 9, **caractérisé en ce qu'**il est prévu une butée de profondeur de piqûre (44) sur laquelle le chariot de piqûre (14) est appliqué en atteignant une profondeur de piqûre (tE) définie et le chariot de piqûre (14) est pressé contre ladite butée pendant la course d'injection, lors de laquelle le poussoir d'actionnement (70) est déplacé par rapport au reste de l'appareil d'injection (8) dans la direction d'injection (R) jusqu'à une position terminale, et d'un temps d'arrêt suivant à cela,
dans lequel le poussoir d'actionnement (70) est précontraint dans une position terminale dans la direction d'injection (R) pendant le temps d'arrêt et l'écrou de transport (32) est espacé du dispositif d'actionnement (12).

11. Dispositif d'injection selon la revendication 10, **caractérisé en ce qu'**il est prévu un capteur (46) pour détecter la position terminale, au moyen duquel un minuteur réglable est commutable, minuteur avec lequel la durée du temps d'arrêt peut être réglée.

12. Dispositif d'injection selon la revendication 10 ou 11, **caractérisé en ce que** la butée de profondeur de piqûre (44) peut être ajustée par rapport au boîtier support (4).

13. Dispositif d'injection selon l'une des revendications 5 à 12, **caractérisé en ce que** des moyens de verrouillage (50) sont prévus entre le chariot de piqûre (14) et le chariot d'injection (16), lesdits moyens de verrouillage pouvant être déplacés entre une position de verrouillage, dans laquelle un engagement par imbrication de formes est réalisé entre le chariot de piqûre (14) et le chariot d'injection (16), et une position de relâchement, dans laquelle le chariot de piqûre (14) et le chariot d'injection (16) peuvent être translatés relativement l'un par rapport à l'autre, les moyens de verrouillage (50) pouvant être ajustés automatiquement entre une position de relâchement et une position de verrouillage au moyen d'un moyen de commande côté boîtier en fonction de la position du dispositif d'actionnement (12).

14. Dispositif d'injection selon la revendication 13, **caractérisé en ce que** les moyens de verrouillage (50) comprennent une bascule (52) pouvant pivoter sur le chariot d'injection (16), la bascule comprenant un premier bras (54), qui peut être amené par imbrication de formes dans la position de verrouillage avec le chariot de piqûre (14), et un deuxième bras (58), par l'intermédiaire duquel la bascule (52) peut être pivoté par rapport au boîtier support (4).

15. Dispositif d'injection selon la revendication 14, **caractérisé en ce que** le moyen de commande comprend un premier élément de déviation (48a) côté boîtier, par lequel le deuxième bras (58) peut être dévié en atteignant une position prédéterminée du chariot de piqûre (14) pendant la course de piqûre, et **en ce que** la bascule (52) peut être pivotée de la position de verrouillage à la position de relâchement.

16. Dispositif d'injection selon la revendication 14 ou 15, **caractérisé en ce qu'**il est prévu un deuxième élément de déviation (48b) côté boîtier, par lequel le deuxième bras (58) peut être dévié en atteignant une position de sortie du chariot de piqûre (14) pendant la course de retrait, et **en ce que** la bascule (52) peut être pivotée de la position de verrouillage à la position de relâchement.

17. Dispositif d'injection selon la revendication 15 ou 16, **caractérisé en ce que** le deuxième bras (58) est précontraint contre le boîtier support (4).

18. Dispositif d'injection selon la revendication 13, **caractérisé en ce que** les moyens de verrouillage (50) comprennent un coulisseau (78) maintenu mobile dans un creux continu du chariot de piqûre (14), le coulisseau étant relié par imbrication de formes avec le chariot d'injection (16) au moyen d'un contour côté boîtier servant de moyen de commande, en fonction de la position du chariot de piqûre (14).

19. Dispositif d'injection selon l'une des revendications 13 à 18, **caractérisé en ce que** la butée de profondeur de piqûre (44) est formée par une pièce formée (40) montée de manière ajustable sur le boîtier support (4), pièce sur laquelle sont aussi formés au moins partiellement les éléments de déviation (48a, 48b).

20. Dispositif d'injection selon la revendication 19, **caractérisé en ce que** la pièce formée (40) peut être translatée au moyen d'une vis de réglage (42) accessible de l'extérieur du boîtier support (4) et s'appuyant contre ladite pièce.
